# EUROPEAN PATENT SPECIFICATION

(11) **EP 3 089 714 B1**
(45) Date of publication and mention of the grant of the patent: **18.03.2020**
(21) Application number: 14784384.1
(22) Date of filing: 15.07.2014
(51) Int. Cl.: A61F 9/007, A61B 17/3201, A61B 17/3211, A61B 17/00

(54) **A BLADE-SCISSORS FOR VITREORETINAL SURGERIES**
KLINGE-SCHERE FÜR DIE VITRORETINALE CHIRURGIE
CISEAUX-LAMES POUR CHIRURGIE VITRÉO-RÉTINIENNE

(30) Priority: 26.11.2013 TR 201313730
(43) Date of publication of application: 09.11.2016
(73) Proprietor: Yetik, Hüseyin, 34158 Istanbul (TR)
(72) Inventor: Yetik, Hüseyin, 34158 Istanbul (TR)
(74) Representative: Hertin und Partner Rechts- und Patentanwälte PartG mbB
(86) International application number: PCT/TR2014/000261
(87) International publication number: WO 2015/080677

(56) References cited:
- EP-A2- 0 003 668
- WO-A1-2011/154086
- CN-U- 202 027 670
- CN-U- 202 365 868
- CN-Y- 201 208 283
- SU-A1- 1 181 642
- US-A1- 2013 218 159

## Description

### TECHNICAL FIELD

The invention relates to cutting apparatus used in eye surgery.

The invention relates particularly to the embodiment used for cleaning the membranes on the surface of the retina and inside the vitreous during vitreoretinal surgical operations of the eye.

### PRIOR ART

Today, two separate tools are used to remove the membranes from the surface of the retina during vitreoretinal surgical operations.

One of these tools is the vitrectomy scissors. The scissors which are used for cleaning the membranes on the surface of the retina and inside the vitreous act only as scissors. That is to say, the region to be cut (adhered area, connection point, membrane section, etc.) is squeezed between two pallets of the scissors and the portion there between is cut by sliding the pallets on each other; namely, by snipping. From side view, the blades (legs) of the scissors can have straight or conformal form, that is to say concave form. The reason of being conformal (concave) is to adapt to the concavity of the inner wall of the eye. That is to say, the sharp area is between two blades. When they are closed, on the other hand, two edges thereof are blunt. It can be totally straight in alternative embodiments. However, the edges of the straight scissors are also blunt when they are closed.

Second one of these tools is MVR (micro-vitreoretinal) (in another word, spear) blades. Both edges thereof are sharp. MVR is first used to enter the highly closed adhered areas where the vitrectomy scissors cannot enter. An incision is created with sharp left and right edges. Then, the remaining connections are cut with the scissors. That is to say, by the help of this blade two edges of which are sharp unlike the scissors, the potential space between the retina and the membrane is incised to create a space for the scissors to process and then the blade is removed from the eye and the operation is continued with the scissors.

During the surgical practice, the surgeon has to exchange these two tools continuously. During this exchanging process, it is necessary to go in and out the eye continuously. Not only leads this to time loss but the eye subject to the surgical practice goes through trauma more frequently as well.

In conclusion, due to the abovementioned drawbacks and inadequacy of the existing solutions with respect to the subject matter, it is deemed necessary to make improvements or developments in cutting apparatus used in eye surgery differently from the known methods.

CN202365868 U discloses medical scissors comprising legs with inner and outer sharp edges.

### OBJECTS OF THE INVENTION

The object of the invention, being inspired by the existing solutions, is to eliminate the abovementioned drawbacks regarding the cutting apparatus used in eye surgery and to improve said cutting apparatus.

The main object of the invention is to develop a single instrument comprising the functions of two different instruments which enable to cut the membranes on the surface of the retina and inside the vitreous and thus cleaning the same during vitreoretinal eye surgical operations.

An object of the invention is to save on time and cost by presenting two separate cutting apparatus in a single embodiment.

Another object of the invention is to provide convenience for the surgeons during the surgical procedure by presenting a single apparatus.

Yet another object of the invention is to ensure that the eye being operated make it through the operation with minimum trauma during the surgical procedure, and thus to shorten the recovery period of the patient.

To achieve the abovementioned objects, a blade-scissor according to claim 1 is provided. Further embodiments are provided in the dependent claims. The blade-scissors is used for cleaning the membranes on the surface of the retina and inside the vitreous during vitreoretinal eye surgical operations and comprises legs and sharp inner edges, wherein said legs comprise sharp outer edge to act as a blade when closed.

Since the invention does not present novelty with respect to the handle which generates and transmits the mechanical power providing motion for the cutting pallets, the handle is the same as the existing vitreoretinal surgical scissors.

The invention only covers the development of the functioning cutting parts.

### FIGURES FACILITATING THE UNDERSTANDING OF THE INVENTION

All the structural and characteristic features and all the advantages of the invention will be more clearly understood thanks to the following figures and detailed description composed with reference to these figures and for this reason, it is necessary that the evaluation be done by taking into consideration these figures and detailed description.

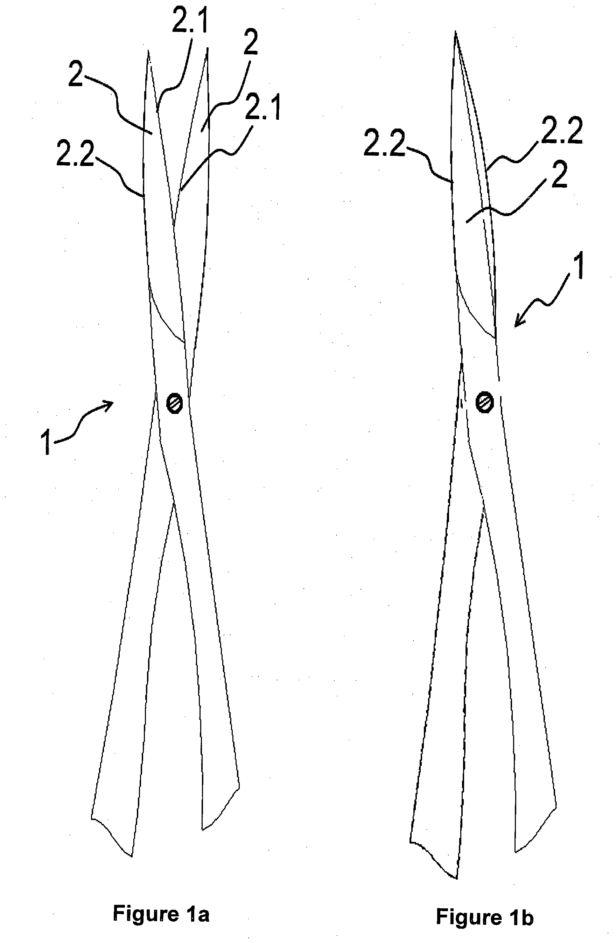

**Figure 1a** is the view of the blade-scissors embodiment according to the invention when opened.
**Figure 1b** is the view of the blade-scissors embodiment according to the invention when it is closed and takes the form of a blade.

### DESCRIPTION OF THE PART REFERENCES

1. Cutting Apparatus
2. Legs
   2.1 Sharp inner edges
   2.2 Sharp outer edges

The drawings do not need to be scaled necessarily and the details that are not necessary for the understanding of the present invention may have been ignored. Apart from this, the elements that are at least substantially identical or that have at least substantially identical functions are shown with same numbers.

### DETAILED DESCRIPTION OF THE INVENTION

Within this detailed description, the preferred embodiments of the blade-scissors according to the invention are disclosed only for the better understanding of the invention.

In the blade-scissors (1) embodiment according to the invention, the functions of a scissors and a blade having sharp edges at both sides are presented in a single apparatus. The same apparatus is used both for incising the potential space between the retina and the membrane, and for cutting the strong connections remained there between by snipping. Thus, the surgical procedure takes a shorter time. Besides, there is no continuous apparatus exchange during the operation, and for this reason, the eye is not traumatized more frequently.

The main function of the blade-scissors (1) embodiment according to the invention is to act as a scissors when opened and as a blade when closed. When the blade scissors (1) is kept closed, it can function as a sharp-pointed blade (as an MVR blade) which comprises sharp inner edges (2.1) and sharp outer edges (2.2).

Moreover, it is possible to provide property of a forceps by adding a third leg (2) thereto. In this case, a single apparatus can have the characteristics of a blade, scissors and forceps at the same time.

## Claims

1. A blade-scissor (1) for cleaning the membranes on the surface of the retina and inside the vitreous during vitreoretinal eye surgical operations wherein the blade-scissor (1) comprises:
a pair of legs (2)
wherein said pair of legs comprises sharp inner edges (2.1) and sharp outer edges (2.2),
wherein the sharp inner edges (2.1) and the sharp outer edges (2.2) form a sharp-pointed blade when the blade-scissor (1) is in a closed position, such that the sharp-pointed blade allows to incise a potential space between the retina and the membranes, and
wherein the blade-scissor (1) is adapted as a scissor, when the blade-scissor (1) is in an open position such that the sharp inner edges (2.1) allow to cut remaining connections between membrane and the retina.

2. The blade-scissor (1) of claim 1, wherein a pointing head of each of the pair of legs (2) comprising the sharp inner edges (2.1) and the sharp outer edges (2.2) forms a sharp point end.

3. The blade-scissor (1) of claim 1, wherein both side edges of the pair of legs are sharpened to form the sharp inner edges (2.1) and the sharp outer edges (2.2).

4. The blade scissor (1) according to claim 1, wherein the blade scissor (1) comprises a third leg, which allows in combination with the pair of legs (2) to form a forceps.

## Patentansprüche

1. Klingenschere (1) zum Reinigen der Membranen auf der Oberfläche der Netzhaut und innerhalb des Glaskörpers während vitreoretinalen Augenoperationen, wobei die Klingenschere (1) umfasst:
ein Paar Schenkel (2)
wobei das Paar Schenkel scharfe Innenkanten (2.1) und scharfe Außenkanten (2.2) umfasst,
wobei die scharfen Innenkanten (2.1) und die scharfen Außenkanten (2.2) eine scharfspitzige Klinge bilden, wenn sich die Klingenschere (1) in einer geschlossenen Position befindet, sodass die scharfspitzige Klinge ermöglicht, einen möglichen Raum zwischen der Netzhaut und den Membranen einzuschneiden, und
wobei die Klingenschere (1) als eine Schere ausgebildet ist, wenn sich die Klingenschere (1) in einer offenen Position befindet, sodass die scharfen Innenkanten (2.1) ermöglichen, verbleibende Verbindungen zwischen Membran und der Netzhaut zu schneiden.

2. Klingenschere (1) nach Anspruch 1, wobei ein spitzer Kopf von jedem des Paars Schenkel (2), das die scharfen Innenkanten (2.1) und die scharfen Außenkanten (2.2) umfasst, ein scharfes Spitzenende bildet.

3. Klingenschere (1) nach Anspruch 1, wobei beide Seitenkanten des Paars Schenkel geschärft sind, um die scharfen Innenkanten (2.1) und die scharfen Außenkanten (2.2) zu bilden.

4. Klingenschere (1) nach Anspruch 1, wobei die Klingenschere (1) einen dritten Schenkel umfasst, der in Kombination mit dem Paar Schenkel (2) das Bilden einer Pinzette ermöglicht.

## Revendications

1. Ciseaux à lames (1) pour le nettoyage des membranes sur la surface de la rétine et à l'intérieur de l'humeur vitrée durant des opérations chirurgicales vitréo-rétiniennes dans lesquels les ciseaux à lames (1) comprennent :
une paire de jambes (2)
dans lesquels ladite paire de jambes comprend des bords intérieur affûtés (2.1) et des bords extérieurs affûtés (2.2),
dans lesquels les bords intérieurs affûtés (2.1) et les bords extérieurs affûtés (2.2) forment une lame à pointe affûtée lorsque les ciseaux à lames (1) se trouvent dans une position fermée, de telle sorte que la lame à pointe affûtée permet l'incision d'un espace potentiel entre la rétine et les membranes, et
dans lesquels les ciseaux à lames (1) sont adaptés en tant que ciseaux, lorsque les ciseaux à lames (1) se trouvent dans une position ouverte de telle sorte que les bords intérieurs affûtés (2.1) permettent de couper les liaisons restantes entre la membrane et la rétine.

2. Ciseaux à lames (1) selon la revendication 1, dans lesquels une tête en pointe de chacune de la paire de jambes (2) comprenant les bords intérieurs affûtés (2.1) et les bords extérieurs affûtés (2.2) forme une extrémité à pointe affûtée.

3. Ciseaux à lames (1) selon la revendication 1, dans lesquels les deux bords latéraux de la paire de jambes sont affûtés pour former les bords intérieurs affûtés (2.1) et les bords extérieurs affûtés (2.2).

4. Ciseaux à lames (1) selon la revendication 1, dans lesquels les ciseaux à lames (1) comprennent une troisième jambe, qui permet en combinaison avec la paire de jambes (2) de former un forceps.
